(19)

Europäisches
Patentamt
European
Patent Office
Office européen
des brevets

(11) **EP 4 248 984 A1**

(12) **EUROPEAN PATENT APPLICATION**
published in accordance with Art. 153(4) EPC

(43) Date of publication:
**27.09.2023 Bulletin 2023/39**

(21) Application number: **21882930.7**

(22) Date of filing: **22.10.2021**

(51) International Patent Classification (IPC):
*A61K 38/08* (2019.01)       *A61K 38/03* (2006.01)
*A61P 21/00* (2006.01)       *C07K 7/06* (2006.01)
*C12N 15/12* (2006.01)

(52) Cooperative Patent Classification (CPC):
**A61K 38/03; A61K 38/08; A61P 21/00; C07K 7/06; C07K 14/435**

(86) International application number:
**PCT/JP2021/039133**

(87) International publication number:
**WO 2022/085791 (28.04.2022 Gazette 2022/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.10.2020 JP 2020178486**

(71) Applicant: **OSAKA UNIVERSITY**
**Suita-shi**
**Osaka 565-0871 (JP)**

(72) Inventors:
• **TANAKA Susumu**
  **Suita-shi, Osaka 565-0871 (JP)**
• **HAMADA Yoshinosuke**
  **Suita-shi, Osaka 565-0871 (JP)**
• **KOGO Mikihiko**
  **Suita-shi, Osaka 565-0871 (JP)**
• **YAMAMOTO Hirofumi**
  **Suita-shi, Osaka 565-0871 (JP)**

(74) Representative: **Wasner, Marita**
**Niizuma Wasner GmbH**
**Patentanwaltskanzlei**
**Postfach 278**
**4125 Riehen (CH)**

Remarks:
The complete document including Reference Table(s) and the Sequence Listing(s) can be downloaded from the EPO website

(54) **MUSCULAR FUNCTION IMPROVING AGENT FOR SKELETAL MUSCLE OF WHICH FUNCTION IS DEGRADED DUE TO AGING**

(57)      [Problem] The present invention addresses the problem of providing a muscular function improving agent for suppressing or ameliorating the degradation of muscular functions due to aging. [Solution] Provided is a muscular function improving agent for suppressing or ameliorating the degradation of functions of skeletal muscle due to aging, the muscular function improving agent containing, as an active ingredient, at least one peptide selected from among the following (1)-(3), or a salt thereof: (1) a peptide consisting of the amino acid sequence represented by SEQ ID NO: 1; (2) a peptide which is the fragment of human osteopontin, and in which the C-terminal amino acid sequence is the amino acid sequence represented by SEQ ID NO: 1; and (3) a peptide which consists of an amino acid sequence obtained by substituting, adding, or deleting one or more amino acids in the amino acid sequence of the peptide in (1) or (2), and has the action of suppressing or ameliorating the degradation of functions of skeletal muscle due to aging.

Fig. 1

**EP 4 248 984 A1**

## Description

### TECHNICAL FIELD

This invention relates to an agent for improving muscle function of skeletal muscles that have deteriorated due to aging.

### BACKGROUND

Among sarcopenia, which is the aging of skeletal muscle, treatment for primary sarcopenia mainly caused by aging primarily consists of nutritional therapy such as protein and amino acid intake and exercise therapy (rehabilitation) to prevent muscle weakness, and no effective therapeutic agents have been discovered. Cell migration and cell differentiation originating from the activity of muscle satellite cells, which are progenitor cells, are essential for skeletal muscle regeneration, but it has been reported that in sarcopenia, the cell number of muscle satellite cells decreases with age and the proliferative function of muscle satellite cells declines (Non-Patent Document 1). However, the mechanism and cause of sarcopenia have not yet been clarified. The number of patients with sarcopenia is increasing year by year, and the development of a therapeutic agent that is effective in inhibiting the progression of the disease will lead to improvements in ADL and QOL and will also contribute to the reduction of financial resources of social security and the "extension of healthy life expectancy," an important national policy, and will have a significant economic impact.

The usefulness of cell therapy, growth factor therapy, and gene therapy using microRNA (miRNA) has been reported as conventional technologies effective for skeletal muscle regeneration (Non-Patent Documents 2-4). However, due to problems such as spontaneous malignant transformation of stem cells, limited administration methods, instability of effective concentration in vivo, immune response, off-target effects of therapeutic miRNA, and high cost in manufacturing and quality control, these technologies have not yet been applied clinically.

The inventors have already shown that a peptide (hereinafter referred to as "SV peptide") consisting of 7 amino acids (SVVYGLR; SEQ No.1) present in osteopontin (OPN), a type of extracellular matrix, has multiple functions such as promoting angiogenesis, type III collagen secretion, and differentiation of fibroblasts to myofibroblasts (Non-Patent Documents 5, 6, and Patent Documents 1-4). SV peptides have a low molecular weight and low antigenicity, and are safer in biological applications than conventional techniques. Local administration of SV peptide in animal models of injured skeletal muscle promotes muscle tissue regeneration, and morphologically, the diameter of regenerated muscle fibers was significantly higher, proving that SV peptide is a potent substance for the regeneration of skeletal muscle function (Non-Patent Document 7 and Patent Document 5).

### PRIOR ART

### PATENT DOCUMENTS

[Patent Document 1] WO2003/030925
[Patent Document 2] WO2008/026634
[Patent Document 3] WO2012/172887
[Patent Document 4] WO2016/084935
[Patent Document 5] WO2018/230535

### NON-PATENT DOCUMENTS

[Non-Patent Document 1] Machida, S. and Booth, FW. (2004). "Regrowth of skeletal muscle atrophied from inactivity," Med Sci Sports Exerc., Jan;36(1):52-9.
[Non-Patent Document 2] Miyagawa, S. and Sawa, Y (2018). "Building a new strategy for treating heart failure using Induced Pluripotent Stem Cells" J Cardiol., Dec;72(6):445-448.
[Non-Patent Document 3] Baoge, L. et al. (2012). "Treatment of Skeletal Muscle Injury: A Review" ISRN Orthop. 689012.
[Non-Patent Document 4] Nakasa, T. et al., (2010). "Acceleration of muscle regeneration by local injection of muscle-specific microRNAs in rat skeletal muscle injury model" J Cell Mol Med., Oct;14(10):2495-505.
[Non-Patent Document 5] Hamada, Y (2003). "Angiogenic activity of osteopontin-derived peptide SVVYGLR" Biochem Biophys Res Commun. Oct 10;310(1):153-7.
[Non-Patent Document 6] Hamada, Y (2007). "Synthetic osteopontin-derived peptide SVVYGLR can induce neovascularization in artificial bone marrow scaffold biomaterials" Dent Mater J. Jul;26(4):487-92.
[Non-Patent Document 7] Tanaka, S. (2019) "Osteopontin-derived synthetic peptide SVVYGLR has potent utility in the functional regeneration of oral and maxillofacial skeletal muscles" Peptides. Jun; 116:8-15.

## SUMMARY OF INVENTION

### PROBLEM TO BE SOLVED

The present invention is to provide a muscle function improving agent that inhibits or improves muscle dysfunction due to aging.

MEANS TO SOLVE THE PROBLEM

Skeletal muscle is considered to be a highly regenerative tissue by nature, but its regenerative capacity declines with age. One of the causes of sarcopenia is thought to be the inability of muscle regeneration to fully compensate for muscle damage due to injury or other causes.

Activation of muscle satellite cells by muscle injury causes cell proliferation and differentiation into myoblasts. Originally, muscle satellite cells are multipotent, and their activation and expression of the MyoD gene determine their differentiation into myoblasts. Myoblasts proliferate to ensure the number of cells required for muscle regeneration. At the same time, the expression of a downstream gene, Myogenin gene, is induced, which is involved in the differentiation and maintenance of myoblasts into myotubular cells, and then mature muscle cells form. Myoblasts undergo cell fusion with myofibers to promote the regeneration of muscle tissue. Here, some myoblasts reenter the quiescent phase of the cell cycle and revert to muscle satellite cells, which is thought to regulate the number of muscle satellite cells intrinsic to skeletal muscle.

However, muscle satellite cells are thought to decrease cell number and self-renewal capacity due to endogenous changes in the cells with aging and exogenous changes such as a decrease in growth factors. This decreased regenerative muscle capacity is thought to be one of the factors in the development of sarcopenia.

The inventors have confirmed that local injection of SV peptide into the muscles of the lower limbs of aging-accelerated model mice can inhibit or improve the decline in muscle function associated with aging. The present invention was completed based on this finding and includes the following aspects :
One aspect of this invention relates to

[1] a muscle function-improving agent for inhibiting or improving functional decline of skeletal muscles due to aging, comprising the peptide selected from the following (1) to (3) or a salt thereof as an active ingredient:

(1) a peptide consisting of an amino acid sequence shown in SEQ ID No. 1
(2) a fragment of human osteopontin, wherein the C-terminal amino acid sequence of the fragment is the amino acid sequence shown in SEQ ID No. 1, and
(3) a peptide consisting of an amino acid sequence that one to several amino acids are deleted, substituted, or added in the amino acid sequence of the peptide in (1) or (2) above and having the effect of inhibiting or improving the decrease in the number of muscle satellite cells in skeletal muscle associated with aging.

In one embodiment, the muscle function-improving agent of the present invention is characterized by the following:
the muscle function-improving agent according to [1] above,

wherein the peptide consisting of an amino acid sequence that one to several amino acids are deleted, substituted, or added in the amino acid sequence of the peptide in (1) and having the effect of inhibiting or improving the decrease in the number of muscle satellite cells in skeletal muscle associated with aging is a peptide consisting of the amino acid sequence of any of the following (I) to (IV),
wherein the peptide consisting of an amino acid sequence that one to several amino acids are deleted, substituted, or added in the amino acid sequence of the peptide in (2) and having the effect of inhibiting or improving the decrease in the number of muscle satellite cells in skeletal muscle associated with aging is a fragment of human osteopontin, the C-terminal amino acid sequence of the fragment being a peptide consisting of the amino acid sequence of any of the following (I) to (IV),

$$X_1-X_2-Val-(Tyr/Phe/Trp)-X_5-X_6-X_7 \quad (I)$$

(In the formula, $X_1$, $X_2$, $X_5$, $X_6$, and $X_7$ represent any amino acid residue, identically or differently)

$$X_1-X_2-Val-(Tyr/Phe/Trp)-X_5-X_6 \quad (II)$$

(In the formula, $X_1$, $X_2$, $X_5$, and $X_6$ represent any amino acid residue, identically or differently)

$$X_2-Val-(Tyr/Phe/Trp)-X_5-X_6-X_7 \quad (III)$$

(In the formula, $X_2$, $X_5$, $X_6$, and $X_7$ represent any amino acid residue, identically or differently)

$$X_1-X_2-Val-(Tyr/Phe/Trp)-X_5-X_6-X_7-X_8 \quad (IV)$$

(In the formula, $X_1$, $X_2$, $X_5$, $X_6$, $X_7$, and $X_8$ represent any amino acid residue, identically or differently) .

Another aspect of the invention relates to
a muscle atrophy inhibitor for inhibiting or improving muscle atrophy of fast muscle fibers and/or slow muscle fibers due to aging, comprising at least one peptide selected from the following (1) to (3) or a salt thereof as an active ingredient:

(1) a peptide consisting of an amino acid sequence shown in SEQ ID No. 1
(2) a fragment of human osteopontin, wherein the C-terminal amino acid sequence of the fragment is the amino acid sequence shown in SEQ ID No. 1, and
(3) a peptide consisting of an amino acid sequence that one to several amino acids are deleted, substituted, or added in the amino acid sequence of the peptide in (1) or (2) above and having the effect of inhibiting or improving muscle atrophy of fast muscle fibers and/or slow muscle fibers due to aging.

In one embodiment, the muscle atrophy inhibitor of the present invention is characterized by the following:
the muscle atrophy inhibitor according to [3] above,

wherein the peptide consisting of an amino acid sequence that one to several amino acids are deleted, substituted, or added in the amino acid sequence of the peptide in (1) and having the effect of inhibiting or improving muscle atrophy of fast muscle fibers and/or slow muscle fibers due to aging is a peptide consisting of the amino acid sequence of any of the following (I) to (IV),
wherein the peptide consisting of an amino acid sequence that one to several amino acids are deleted, substituted, or added in the amino acid sequence of the peptide in (2) and having the effect of inhibiting or improving muscle atrophy of fast muscle fibers and/or slow muscle fibers due to aging is a fragment of human osteopontin, the C-terminal amino acid sequence of the fragment being a peptide consisting of the amino acid sequence of any of the following (I) to (IV),

$$X_1-X_2-Val-(Tyr/Phe/Trp)-X_5-X_6-X_7 \quad (I)$$

(In the formula, $X_1$, $X_2$, $X_5$, $X_6$, and $X_7$ represent any amino acid residue, identically or differently)

$$X_1-X_2-Val-(Tyr/Phe/Trp)-X_5-X_6 \quad (II)$$

(In the formula, $X_1$, $X_2$, $X_5$, and $X_6$ represent any amino acid residue, identically or differently)

$$X_2-Val-(Tyr/Phe/Trp)-X_5-X_6-X_7 \quad (III)$$

(In the formula, $X_2$, $X_5$, $X_6$, and $X_7$ represent any amino acid residue, identically or differently)

$$X_1-X_2-Val-(Tyr/Phe/Trp)-X_5-X_6-X_7-X_8 \quad (IV)$$

(In the formula, $X_1$, $X_2$, $X_5$, $X_6$, $X_7$, and $X_8$ represent any amino acid residue, identically or differently) .

In one embodiment, the muscle function-improving agent of the present invention is characterized by the following:
(5) the muscle function-improving agent according to [1] or [2] above, wherein the agent is for use in conjunction with the loading of stimuli on the skeletal muscle.

In one embodiment, the muscle function-improving agent of the present invention is characterized by the following:
the muscle function-improving agent according to [5] above, wherein the agent is for preventing or improving the functional decline of the skeletal muscle due to sarcopenia or inclusion body myositis

In one embodiment, the muscle function-improving agent of the present invention is characterized by the following:
the muscle function-improving agent according to [6] above, wherein the loading of the stimulus to the skeletal muscles is exercise therapy.

In one embodiment, the muscle function-improving agent of the present invention is characterized by the following:
a muscle-function improving agent for inhibiting or improving the functional decline of the skeletal muscle, wherein the muscle-function improving agent is for use in conjunction with the loading of stimuli and comprise at least one peptide selected from the following (1) to (3) or a salt thereof as an active ingredient:

(1) a peptide consisting of an amino acid sequence shown in SEQ ID No. 1
(2) a fragment of human osteopontin, wherein the C-terminal amino acid sequence of the fragment is the amino acid sequence shown in SEQ ID No. 1, and
(3) a peptide consisting of an amino acid sequence that one to several amino acids are deleted, substituted, or added in the amino acid sequence of the peptide in (1) or (2) above and having the effect of inhibiting or improving skeletal muscle dysfunction.

## EFFECT OF THE INVENITON

According to the muscle function-improving agent of the present invention, it can improve the decline function of a skeletal muscle due to aging.

## BRIEF DESCRIPTION OF THE DRAWINGS

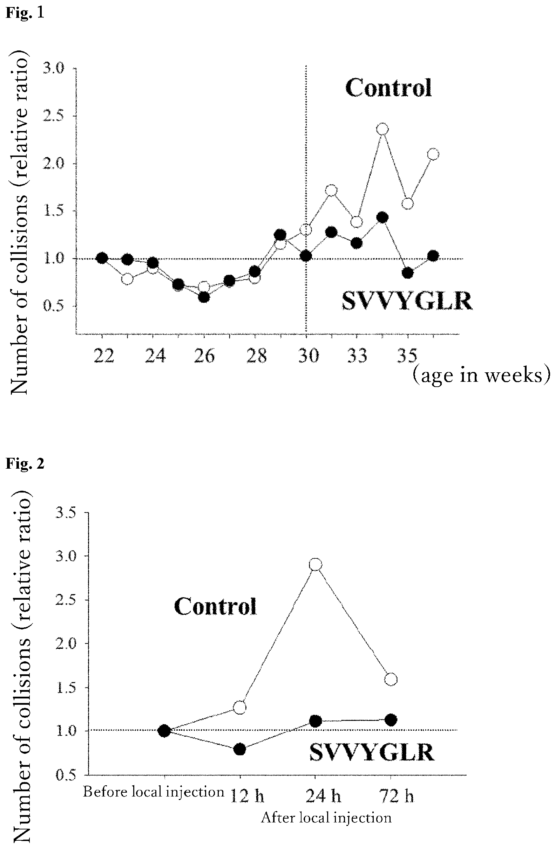

[Fig. 1] Figure 1 shows the number of collisions during treadmill running performed on aging-accelerated model mice from 22 to 35 weeks of age. The treadmill running was performed after local injection of SV peptide into the bilateral lower limb muscles after treadmill execution. The control group represents the group injected with PBS instead of SV peptide.

[Fig. 2] Figure 2 shows the number of collisions during treadmill running performed on aging-accelerated model mice at 36 weeks of age. The treadmill running was performed 12, 24, or 72 hours after the local injection of SV peptide into the bilateral lower limb muscles after treadmill execution. The control group represents the group injected with PBS instead of SV peptide.

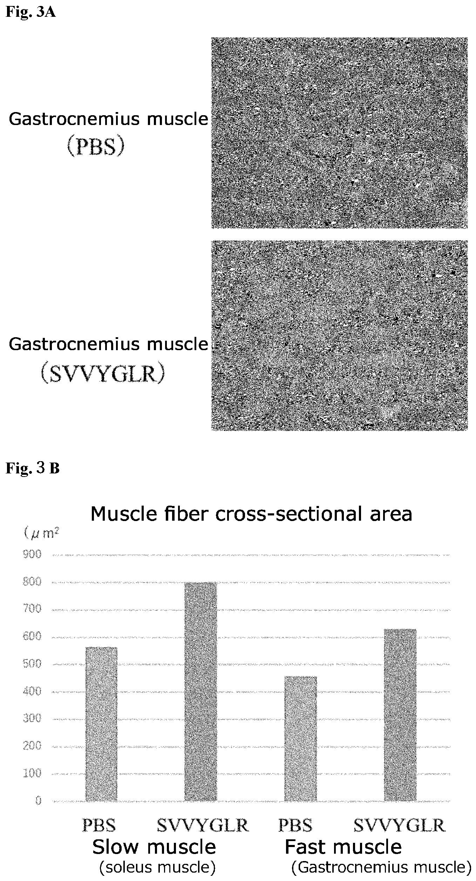

[Fig. 3A] Figure 3A shows the results of HE staining of the gastrocnemius muscle (fast muscle) of aging-accelerated model mice in which SV peptide was localized to the bilateral lower limb muscles after treadmill running from 22 to 40 weeks of age.

[Fig. 3B] Figure 3B shows graphs of the mean cross-sectional area of muscle fibers in the gastrocnemius muscle (fast muscle) and soleus muscle (slow muscle) of aging-accelerated model mice injected with SV peptide in bilateral leg muscles after treadmill running from 22 weeks to 40 weeks of age. The control group represents the group injected with PBS instead of SV peptide.

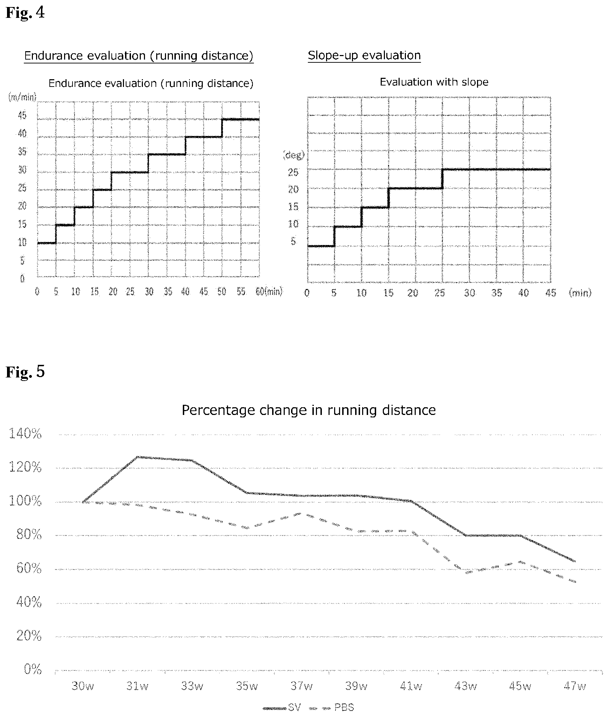

[Fig. 4] Figure 4 shows a graph showing the relationship between elapsed time and belt speed increase employed in the endurance evaluation test in Example 4 below, and a graph showing the relationship between elapsed time and belt slope increase employed in the slope-up evaluation test.

[Fig. 5] Figure 5 shows the percentage change in the mean running distance of the SV group (training performed and SV peptide injected), the SVN group (SV peptide injected), and the PBS group (training performed and PBS injected) in the endurance evaluation test conducted in Example 4 below.

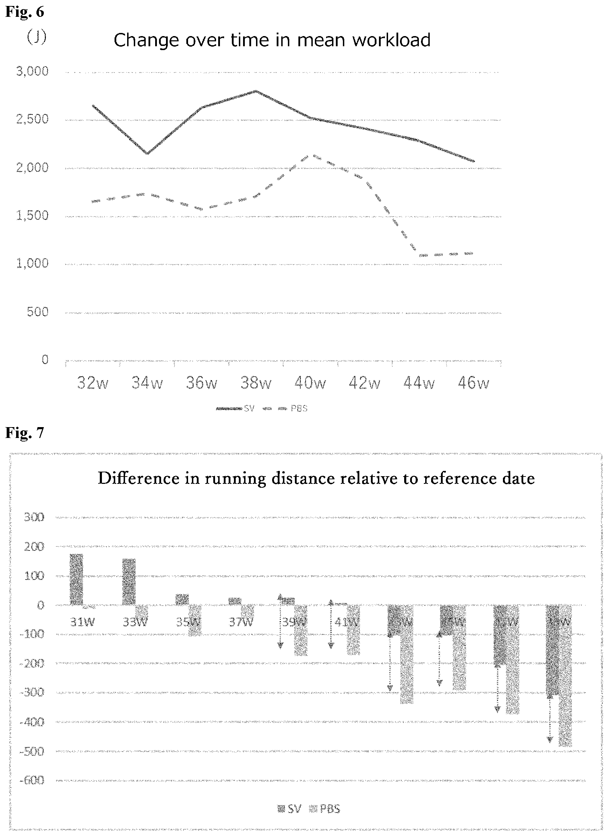

[Fig. 6] Figure 6 shows the changes over time in the mean workload of the SV group (training performed and SV peptide injected), SVN group (SV peptide injected), and PBS group (training performed and PBS injected) in the endurance evaluation test conducted in Example 4 below.

[Fig. 7] Figure 7 is a graph showing the change over time in the mean running distance relative to the reference date of the SV group (training performed and SV peptide injected) and the PBS group (training performed and PBS injected) in the endurance evaluation test conducted in Example 5 below. The arrows in Figure 7 indicate the difference between the SV and PBS groups in the change in mean running distance (indicating the effect of SV administration).

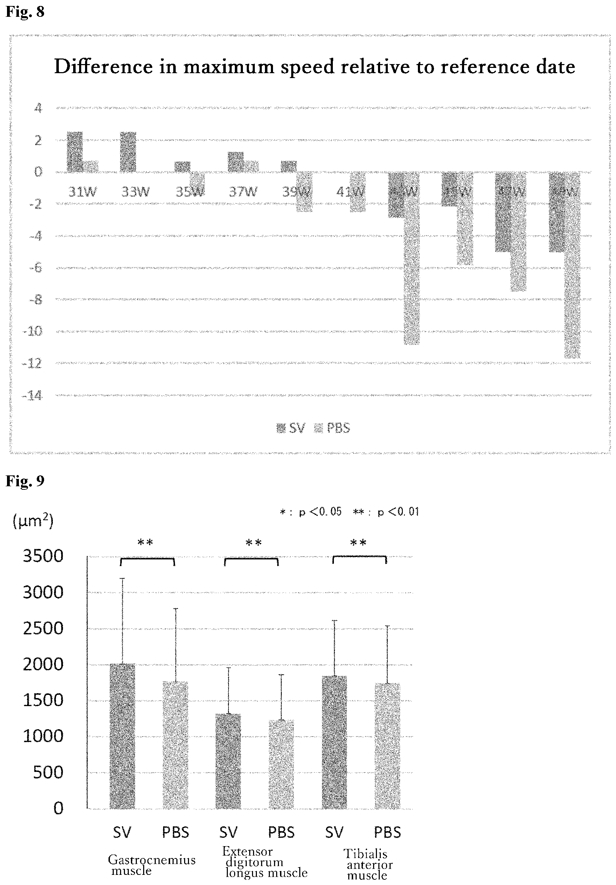

[Fig. 8] Figure 8 is a graph showing the change over time in the mean maximum speed relative to the reference date of the SV group (training performed and SV peptide injected) and the PBS group (training performed and PBS injected) in the endurance evaluation test conducted in Example 5 below.

[Fig. 9] Figure 9 shows a graph of the mean cross-sectional area of muscle fibers in the gastrocnemius, extensor digitorum longus, and tibialis anterior muscles of aging-accelerated model mice injected with SV peptide in bilateral leg muscles after treadmill running from the age of 28 weeks to 51 weeks. The control group represents the group in which PBS was injected instead of SV peptide.

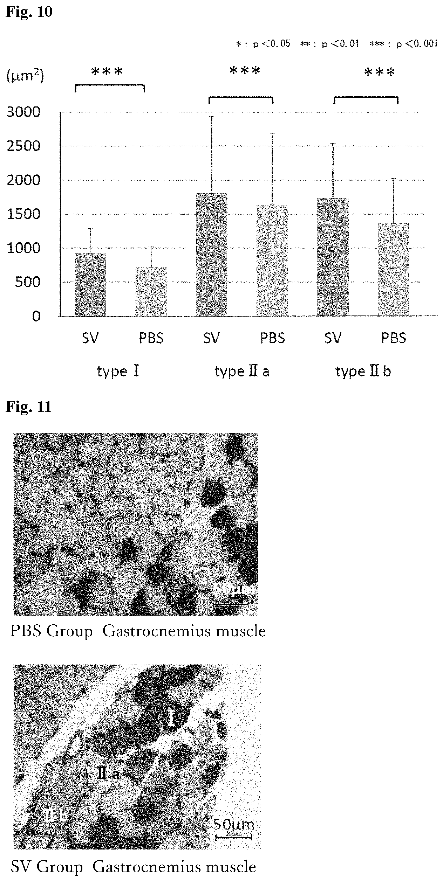

[Fig. 10] Figure 10 shows a graph of the mean cross-sectional area of type I, type IIa, and type IIb muscle fibers in the gastrocnemius muscles of the aging-accelerated model mice injected with SV peptide in the bilateral leg muscles after treadmill running from 28 weeks to 51 weeks of age. The control group represents the group in which PBS was injected instead of SV peptide.

[Fig. 11] Figure 11 shows the results of HE staining of the gastrocnemius muscle (fast muscle) of an accelerated aging model mouse in which SV peptide was localized to the bilateral lower limb muscles after treadmill running from the age of 28 weeks to 51 weeks.

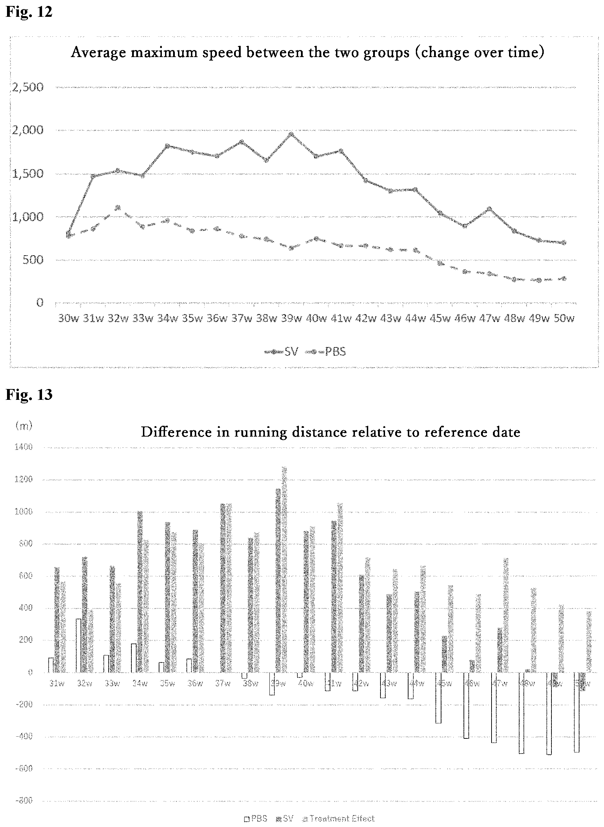

[Fig. 12] Figure 12 is a graph showing the change over time of the mean running distance of the SV group (training performed and SV peptide injected) and the PBS group training performed and PBS injected) in the endurance evaluation test conducted in Example 6 below.

[Fig. 13] Figure 13 is a graph showing the amount of the change over time in the mean running distance relative to a reference date of the SV group (training performed and SV peptide injected) and the PBS group (training performed and PBS injected) in the endurance evaluation test conducted in Example 6 below.

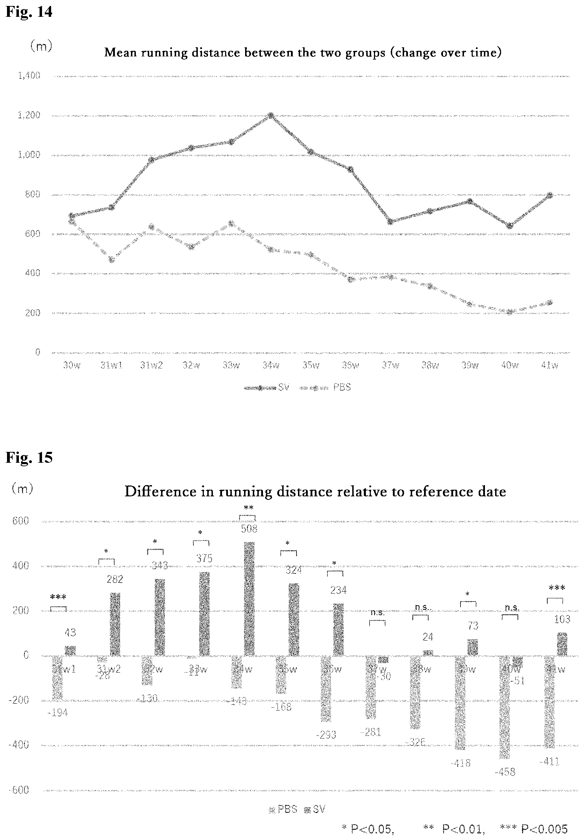

[Fig. 14] Figure 14 is a graph showing the change over time of the mean running distance of the SV group (training performed and SV peptide injected) and the PBS group (training performed and PBS injected) in the endurance evaluation test conducted in Example 7 below.

[Fig. 15] Figure 15 is a graph showing the amount of the change over time in the mean running distance relative to a reference date of the SV group (training performed and SV peptide injected) and the PBS group (training performed and PBS injected) in the endurance evaluation test conducted in Example 7 below.

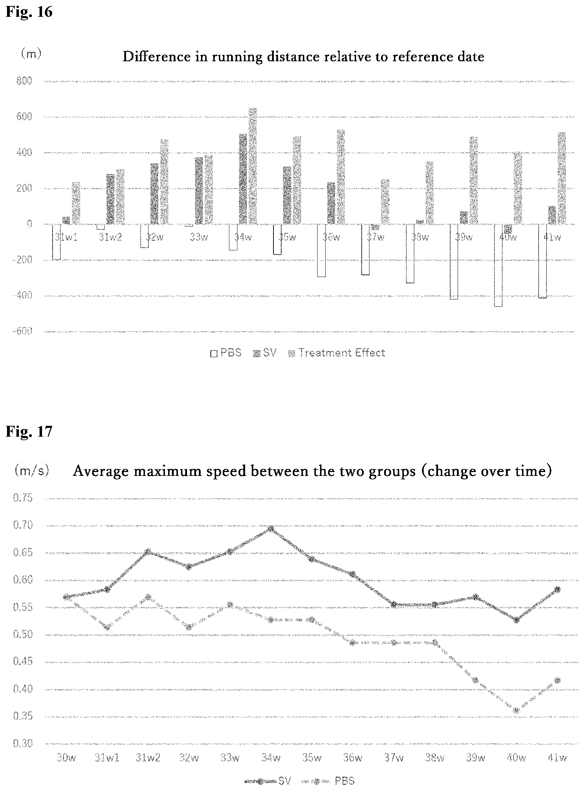

[Fig. 16] Figure 16 is a graph showing the amount of the change over time in the mean running distance relative to a reference date of the SV group (training performed and SV peptide injected) and the PBS group (training performed and PBS injected) in the endurance evaluation test conducted in Example 7 below. In the graph, "treatment effect" is the sum of the absolute difference in mean running distance relative to the reference date of the SV group and the absolute difference in mean running distance relative to the reference date of the PBS group.

[Fig. 17] Figure 17 is a graph showing the change over time of the mean maximum speed of the SV group (training performed and SV peptide injected) and the PBS group (training performed and PBS injected) in the endurance evaluation test conducted in Example 7 below.

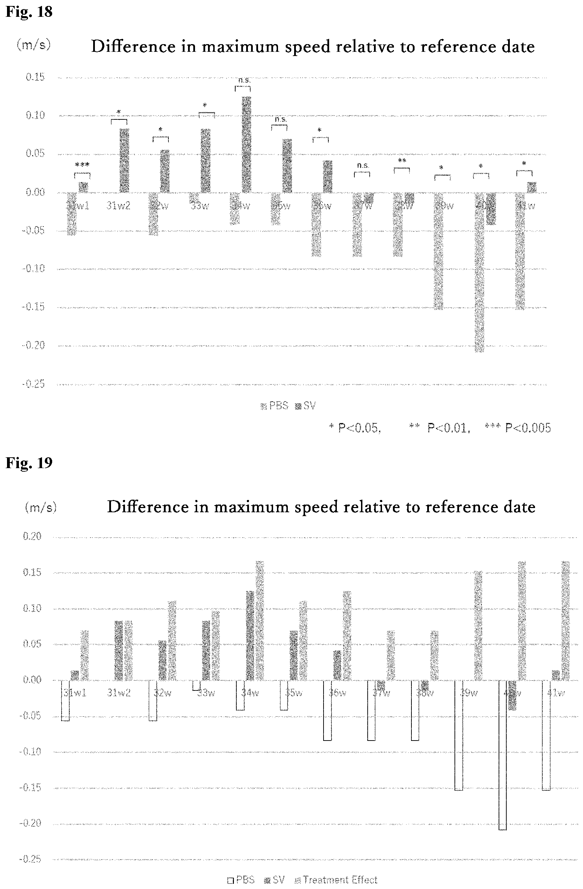

[Fig. 18] Figure 18 is a graph showing the amount of the change over time of the mean maximum speed relative to the reference date of the SV group (training performed and SV peptide injected) and the PBS group (training performed and PBS injected) in the endurance evaluation test conducted in Example 7 below.

[Fig. 19] Figure 19 is a graph showing the amount of the change over time of the mean maximum speed relative to the reference date of the SV group (training performed and SV peptide injected) and the PBS group (training performed and PBS injected) in the endurance evaluation test conducted in Example 7 below. In the graph, "treatment effect" is the sum of the absolute difference in mean running distance relative to the reference date of the SV group and the absolute difference in mean running distance relative to the reference date of the PBS group.

## EMBODIMENTS

The present invention, in an aspect, provides a muscle function-improving agent for inhibiting or improving the functional decline of a skeletal muscle due to aging,
comprising the peptide selected from the following (1) to (3) or a salt thereof as an active ingredient:

(1) a peptide consisting of an amino acid sequence shown in SEQ ID No. 1

(2) a fragment of human osteopontin, wherein the C-terminal amino acid sequence of the fragment is the amino acid sequence shown in SEQ ID No. 1, and

(3) a peptide consisting of an amino acid sequence that one to several amino acids are deleted, substituted, or added in the amino acid sequence of the peptide in (1) or (2) above and having the effect of inhibiting or improving the functional decline of the skeletal muscle due to aging.

The term "functional decline of a skeletal muscle due to aging" refers to a decrease in skeletal muscle function resulting from a decline in physiological function. For example, it means a state in which the function to contract or relax muscle fibers declines along with a decrease in skeletal muscle mass caused by a decrease in the regenerative capacity of cells (myocytes, myoblasts, or myocytes) constituting skeletal muscle due to aging. As long as the "functional decline of skeletal muscle due to aging" is caused by a decline in physiological function, it also includes a state in which the skeletal muscle function has declined due to causes other than aging, such as genetic factors, inactivity, diseases such as organ failure, and nutritional status.

The amino acid sequence of human osteopontin includes, but is not limited to, the amino acid sequence shown in SEQ No.: 11. Human osteopontin contains the amino acid sequence shown in SEQ No.: 1 (SVVYGLR), and thrombin cleaves human osteopontin just after SVVYGLR in the amino acid sequence shown in sequence number 11, resulting in a fragment having the sequence of SVVYGLR at the C-terminal end. A "fragment of human osteopontin" herein refers to such a fragment (SEQ No.: 12) or a portion thereof, having the sequence of SVVYGLR at the C-terminus. Proteins having the same structure and function as human osteopontin can be referred to as human osteopontin and their amino acid sequence is the amino acid sequence of human osteopontin.

The length of the fragment of human osteopontin is not particularly limited, but it is preferred that the total number of amino acid residues be about 170 or less, more preferably about 150 or less, and even more preferably about 100 or less. Furthermore, from the viewpoint of simplicity of handling, manufacturing efficiency, and side effects such as antigenicity, it is preferred that the total number of amino acid residues be about 50 or less, about 30 or less is more preferred, about 20 or less is even more preferred, and about 10 or less is especially preferred.

In this specification, "amino acid sequence that one to several amino acids are deleted, substituted, or added" means an amino acid sequence in which a sufficient number (preferably 10 or less, more preferably 7 or less, more preferably 5, 4, 3, 2, or 1) of amino acids are deleted, substituted (preferably conservative substitution), or added by known methods of producing mutant peptides, such as site-directed mutagenesis. An "amino acid sequence that is at least 80% identical" includes an amino acid sequence that is at least 85%, 90%, 92%, 95%, 96%, 97%, 98% or 99% identical.

Amino acids that are functionally similar to a particular amino acid as a conservative substitution are well known in the art. Examples of properties of amino acid side chains are hydrophobic amino acids (A, I, L, M, F, P, W, Y, V), hydrophilic amino acids (R, D, N, C, E, Q, G, H, K, S, T), and side chains having the following common functional groups or features: aliphatic side chains (G, A, V, L, I, P); hydroxyl group containing side chains (S, T Y); sulfur atom-containing side chains (C, M); carboxylic acid and amide-containing side chains (D, N, E, Q); base-containing side chains (R, K, H); and aromatic-containing side chains (H, F, Y, W). The following eight groups also contain amino acids that are each mutually conservative substitutions: 1) alanine (A), glycine (G); 2) aspartic acid (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M) (see for example Creighton, Proteins 1984).

In one embodiment, a peptide consisting of the amino acid sequence of any of the following (I) to (IV) can be listed as a peptide consisting of an amino acid sequence in which one to several amino acids are substituted, added, or deleted in the amino acid sequence of a peptide consisting of the amino acid sequence shown in SEQ ID No.: 1 and having an effect of inhibiting or improving the functional decline of skeletal muscle due to aging:

$$\mathrm{X_1-X_2-Val-(Tyr/Phe/Trp)-X_5-X_6-X_7 \quad (I)}$$

(In the formula, $X_1$, $X_2$, $X_5$, $X_6$, and $X_7$ represent any amino acid residue, identically or differently)

$$\mathrm{X_1-X_2-Val-(Tyr/Phe/Trp)-X_5-X_6 \quad (II)}$$

(In the formula, $X_1$, $X_2$, $X_5$, and $X_6$ represent any amino acid residue, identically or differently)

$$X_2-Val-(Tyr/Phe/Trp)-X_5-X_6-X_7 \quad (III)$$

(In the formula, $X_2$, $X_5$, $X_6$, and $X_7$ represent any amino acid residue, identically or differently)

$$X_1-X_2-Val-(Tyr/Phe/Trp)-X_5-X_6-X_7-X_8 \quad (IV)$$

(In the formula, $X_1$, $X_2$, $X_5$, $X_6$, $X_7$, and $X_8$ represent any amino acid residue, identically or differently) .

In the preferred embodiment, $X_1$ in the amino acid sequence of any of (I) to (IV) above is serine or a conservatively substituted amino acid thereof, $X_2$ and $X_3$ are valine or a conservatively substituted amino acid thereof, $X_5$ is glycine or a conservatively substituted amino acid thereof, $X_6$ is leucine or a conservatively substituted amino acid thereof, or $X_7$ is arginine or a conservatively substituted amino acid thereof.

A peptide consisting of any of the amino acid sequences (I) to (IV) above may also have several more amino acids at its N-terminus or C-terminus, as long as it acts to inhibit or improve the functional decline of skeletal muscle due to aging.

In another embodiment, a peptide consisting of the amino acids shown in (V) below can be listed as a peptide comprising one to several amino acid substitutions, additions, or deletions in the amino acid sequence of the peptide consisting of the amino acid sequence shown in SEQ ID NO.: 1 and having the effect of inhibiting or improving the functional decline of skeletal muscle due to aging:

$$X_1-Val-Val-(Tyr/Phe/Trp)-X_5-X_6-X_7 \quad (V)$$

In another embodiment, peptides consisting of the amino acids shown in SEQ ID NOs.: 2-10 below can be listed as a peptide comprising one to several amino acid substitutions, additions, or deletions in the amino acid sequence of the peptide consisting of the amino acid sequence shown in SEQ ID NO.: 1 and having the effect of inhibiting or improving the functional decline of skeletal muscle due to aging.

Table 1

| | |
|---|---|
| SWFGLR | (SEQ ID No.: 2 ) |
| SVVWGLR | (SEQ ID No.: 3 ) |
| SVVYGI, | (SEQ ID No.: 4 ) |
| VVYGLR | (SEQ ID No.: 5 ) |
| SVVWGI_ | (SEQ ID No.: 6 ) |
| VVWGLR | (SEQ ID No.: 7 ) |
| VVFGLR | (SEQ ID No.: 8 ) |
| SVVFGL | (SEQ ID No.: 9 ) |
| SVVYGLRG | (SEQ ID No.: 10 ) |

The inventors have confirmed that the angiogenic effect of SVVYGLR (SEQ ID NO.: 1) is maintained in the peptide in which R at the C-terminal of SVVYGLR (SEQ ID NO.: 1) is deleted (SEQ ID NO.: 4), the peptide in which S at the N-terminal is deleted (SEQ ID NO.: 5), and the peptide in which an amino acid is added to the C terminus of SVVYGLR (SEQ ID NO.: 10). The inventors have also confirmed that the angiogenic effect of SVVYGLR (SEQ ID NO.: 1) is maintained in the peptides in which each amino acid other than the fourth tyrosine (Y) in SEQ ID No.: 1 is replaced by an alanine (e.g., SEQ ID Nos.: 2, 3, and 4). The inventors have also confirmed that a fragment of osteopontin having SVVYGLR (SEQ ID No.: 1) at the C-terminus has an effect equivalent to type III collagen production-promoting effect of SVVYGLR (SEQ ID No.: 1) on fibroblasts (see Patent Document 1). It is reasonably analogous that all of the above peptides (1) to (3) have muscle function-improving agent effects.

In one embodiment, a fragment of human osteopontin, comprising one to several amino acid substitutions, additions, or deletions in the amino acid sequence of the peptide whose C-terminal amino acid sequence is the amino acid sequence shown in SEQ ID No.: 1, and having the effect of inhibiting or improving the functional decline of skeletal muscle due to aging include fragments of human osteopontin in which one to several amino acids are substituted, added, or deleted in the C-terminal portion of the amino acid sequence, in the amino acid sequence portion other than the C-terminal portion, or in both thereof amino acid sequence portions.

Amino acids constituting the peptide used as the active ingredient of the muscle function-improving agent of the present invention may have their side chains modified by any substituent. The substituent is not limited but includes, for example, a fluorine atom, a chlorine atom, a cyano group, a hydroxyl group, a nitro group, an alkyl group, a cycloalkyl group, an alkoxy group, and an amino group. Preferably, the benzene ring of tryptophan or phenylalanine is modified by a substituent.

The peptide used as the active ingredient of the muscle function-improving agent of the present invention may have a carboxyl group (-COOH), a carboxylate (-COO-), an amide (-CONH2) or an ester (-COOR) at the C-terminus. Examples of the R moiety in the ester include $C_{1-6}$ alkyl groups such as methyl, ethyl, n-propyl, isopropyl, and n-butyl; $C_{3-8}$ cycloalkyl groups such as cyclopentyl and cyclohexyl; $C_{6-12}$ aryl groups such as phenyl and $\alpha$-naphthyl; and $C_{7-14}$ aralkyl groups such as phenyl-$C_{1-2}$ alkyl groups including benzyl and phenethyl, and $\alpha$-naphthyl-$C_{1-2}$ alkyl groups including $\alpha$-naphthylmethyl. Also included is a pivaloyloxymethyl group, which is widely used in an ester for oral use. Examples of the amide moiety include amides; amides substituted with one or two $C_{1-6}$ alkyl groups; amides substituted with one or two $C_{1-6}$ alkyl groups substituted with a phenyl group; and amides in which a 5- to 7-membered azacyclo alkane containing the nitrogen atom of the amide group is formed. When the peptide of the present invention has a carboxyl group or a carboxylate group at a site other than the C-terminus, these groups may be amidated or esterified. Such modified peptides are encompassed in the scope of the peptide of the present invention.

In the peptide used as the active ingredient of the muscle function-improving agent of the present invention, the N-terminal amino group may be protected by a protecting group (e.g., $C_{1-6}$ acyl groups including a formyl group and a $C_{2-6}$ alkanoyl group such as acetyl, etc.). In said peptide, the substituent in the side chain of an intramolecular amino acid may be protected by an appropriate protecting group (e.g., $C_{1-6}$ acyl groups including a formyl group, and a $C_{2-6}$ alkanoyl group such as acetyl, etc.). Such modified peptides are encompassed in the scope of the peptide of the present invention.

The peptide used as the active ingredient of the muscle function-improving agent of the present invention may be in the form of a salt, and the salt is preferably a pharmaceutically acceptable salt. Examples of the pharmaceutically acceptable salt include salts with acids such as hydrochloric acid, sulfuric acid, phosphoric acid, lactic acid, tartaric acid, maleic acid, fumaric acid, oxalic acid, malic acid, citric acid, oleic acid, palmitic acid, etc.; salts with alkali metals such as sodium and potassium, salts with alkaline earth metals such as calcium, and salts with aluminum hydroxide or carbonate; and salts with triethylamine, benzylamine, diethanolamine, t-butylamine, dicyclohexylamine, arginine, etc.

The peptide used as the active ingredient of the muscle function-improving agent of the present invention or a salt thereof can be produced by a solid phase synthesis method (e.g., the Fmoc method and the Boc method) or a liquid phase synthesis method according to a known ordinary peptide synthesis protocol. Alternatively, a transformant with an expression vector containing a DNA encoding the peptide can be used to produce the peptide. Also, the peptide can be produced by in vitro coupled transcription-translation system.

Subjects of aging skeletal muscles that can be functionally improved by the muscle function-improving agent of the present invention include, for example, skeletal muscles that have developed sarcopenia and/or age-related muscle atrophy or degeneration, skeletal muscles that have developed inclusion body myositis, and muscle atrophy promoted by postoperative bed-ridden conditions such as hip fracture surgery.

Sarcopenia is defined as a decrease in skeletal muscle mass and a decrease in muscle strength or physical function due to aging and other factors. There are several reports on the criteria for determining sarcopenia, which is summarized, for example, in the 2017 edition of the Guidelines for the Treatment of Sarcopenia (published by the Japanese Association on Sarcopenia and Frailty, National Center for Geriatrics and Gerontology). Sarcopenia is classified into primary sarcopenia associated with aging and secondary sarcopenia associated with causes other than aging (including sarcopenia associated with activity such as inactivity; sarcopenia associated with diseases such as organ failure, inflammatory diseases, malignancy, and endocrine disorders; sarcopenia associated with nutrition such as malnutrition). The muscle function-improving agent can be used to treat both primary sarcopenia and secondary sarcopenia.

Examples of skeletal muscle atrophy include, but are not limited to, lower limb muscle atrophy, lower limb muscle disuse atrophy, muscle atrophy, scapulohumeral atrophy, limb muscle atrophy, upper limb muscle atrophy, generalized muscle atrophy, trapezius muscle partial muscle atrophy, degenerative muscle atrophy, disuse syndrome, disuse muscle atrophy, and thenar muscle atrophy.

The muscle function-improving agent of the present invention can be implemented as a pharmaceutical, cosmetic, or food product for improving the function of skeletal muscles that have deteriorated due to aging. In other words, as other aspects of this invention, pharmaceutical compositions, cosmetics, and food and beverage compositions containing the muscle function-improving agent of the present invention can be provided. When implemented as a pharmaceutical or cosmetic product, the present muscle function-improving agent can be formulated by blending a pharmaceutically acceptable carrier or additive agent as appropriate. Specific examples of the dosage form include oral preparations such as tablets, coated tablets, pills, powders, granules, capsules, solutions, suspensions, and emulsions; and parenteral preparations such as injections, microneedle, infusions, suppositories, ointments, and patches. The amount of the carrier or the additive to be used is determined as appropriate based on the range of amount conventionally used in the pharmaceutical field. The carrier or the additive that can be used is not particularly limited, and examples include various carriers such as water, physiological saline, other aqueous solvents, and aqueous or oily bases; and various additives such as excipients, binders, pH adjusters, disintegrants, absorption enhancers, lubricants, colorants, corrigents, and fragrances. When implemented as a food and beverage composition, it can be prepared by appropriately blending with carriers and additives normally used in the food and beverage field.

Examples of the additive that can be blended into tablets, capsules, and the like include binders such as gelatin, cornstarch, tragacanth, and gum arabic; fillers such as crystalline cellulose; bulking agents such as cornstarch, gelatin, and alginic acid; lubricants such as magnesium stearate; sweeteners such as sucrose, lactose, and saccharin; and flavors such as peppermint, Gaultheria adenothrix oil, and cherry. In the case where the unit dosage form is a capsule, a liquid carrier such as fats and oils can be further contained in addition to the above-mentioned ingredients. A sterile composition for injection can be prepared according to the usual pharmaceutical formulation practice, for example, by dissolving or suspending an active substance in a vehicle such as water for injection and a natural vegetable oil such as sesame oil and coconut oil. As an aqueous liquid for injection, for example, physiological saline, an isotonic solution containing glucose and an auxiliary substance (e.g., D-sorbitol, D-mannitol, sodium chloride, etc.), or the like can be used, optionally together with a suitable solubilizer such as alcohols (e.g., ethanol etc.), polyalcohols (e.g., propylene glycol, polyethylene glycol, etc.), and nonionic surfactants (e.g., polysorbate 80™, HCO-50, etc.). As an oily liquid, for example, sesame oil, soybean oil, or the like can be used, optionally together with a solubilizer such as benzyl benzoate and benzyl alcohol. Further, a buffering agent (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, and/or the like may also be added.

The muscle function-improving agent may be implemented as an injection or microneedle for direct administration to the target skeletal muscle or surrounding muscle (skeletal muscle), or as an ointment or patch for application or affixing to the skeletal muscle or surrounding muscle. Microneedle technology is a transdermal formulation in patch form that contains the drug at the tip of a microscopic needle and is applied to the skin to administer the drug to the body. The muscle function-improving agent can be used in combination with known microneedle technology. When provided as an ointment or patch formulation for application or affixing to the skin, it can also be used in combination with known transdermal absorption enhancers or methods for enhancing transdermal absorption (such as transdermal absorption formulations using microemulsion gel or bioabsorbable gel). The muscle function-improving agent may also be in a form in which the active ingredient, peptide, is bound to a carrier. The carrier is not particularly limited, and examples include resins for use in artificial organs etc., and biopolymers such as proteins. In a particularly preferable embodiment, the agent of the present invention is in the form of a bioabsorbable gel entrapping the active ingredient peptide.

Known bioabsorbable hydrogels are preferably used as the bioabsorbable gel. Specific examples include "MedGEL (trade name)", a hydrogel for sustained-release manufactured by MedGEL Corporation. This product is a water-insoluble material formed by cross-linking of gelatin and can entrap a peptide via intermolecular interaction including electrostatic interaction between the peptide and the gelatin. When the active ingredient peptide is entrapped in such a gelatin hydrogel and applied to a living body, the gelatin hydrogel is degraded by degrading enzymes such as collagenase secreted from cells. Upon hydrogel degradation, the peptide is gradually released, and the degradation product is absorbed into the living body. The shape of the bioabsorbable gel is not particularly limited, and various shapes may be employed such as a sheet, a disk, a tube, and a particle. The bioabsorbable gel can be applied or affixed to the site of skeletal muscle injury.

The peptide used as the active ingredient of the muscle function-improving agent of the present invention or a salt thereof is safe and less toxic, and therefore can be administered to, for example, humans and other mammals (rats, mice, rabbits, sheep, pigs, cows, cats, dogs, monkeys, etc.).

The dosage varies depending on the site of dysfunction, route of administration, etc. For example, when injected intramuscularly around dysfunctional skeletal muscles in adults, the daily dosage of the active ingredient may be from about 0.00001 to 100 mg, from about 0.00002 to 90 mg, from about 0.00005 to 80 mg, from about 0.0001 to 50 mg, from about 0.01 to 30 mg, from about 0.1 to 20 mg, or from about 0.1 to 10 mg.

The muscle function-improving agent can be administered, for example, daily (once per day, twice per day, three times per day, four times per day, five times per day, six times per day), every two days, every three days, every four days, every five days, every six days, weekly, twice per week, every other week, every three weeks, once per four weeks, monthly, every two months, once every three months, once every four months, once every five months or once every six months.

In one embodiment, the muscle function-improving agent of the present invention can be provided as a muscle function-improving agent for use in combination with a stimulus load to a skeletal muscle functionally declined.

When used in combination with a stimulus load to skeletal muscles, the muscle function-improving agent of the present invention can suppress the functional decline of skeletal muscles more than when the muscle function-improving agent is used alone or when a stimulus load to skeletal muscles is applied alone. In a preferred embodiment, the muscle function-improving agent of the present invention can have an excellent muscle function-improving effect that cannot be obtained when the muscle function-improving agent is used alone or when the stimulation load to skeletal muscles is applied alone.

"Stimulus loading to skeletal muscles" is not limited as long as it is a stimulus loading that elicits muscle contraction and/or relaxation of skeletal muscles. Known stimulus-loading methods can be employed as stimulus loading to skeletal muscles. These include, but are not limited to, stimulus loading by exercise therapy, etc., loading by physiological muscle stimulation methods (e.g., electrical stimulation methods (neuromuscular electrical stimulation: NMES) (Jpn J Rehabil Med 2017; 54:764-767), stimulus loading by vibration (see, for example, Patent 6886559), etc.)

The "exercise load to skeletal muscles with functional decline" is not limited as long as it can suppress or improve the functional decline of skeletal muscles when used in combination with the muscle function-improving agent of the present invention, and is not limited to the following, for example, when targeting patients suffering from sarcopenia, known exercise therapies used for the treatment of sarcopenia can be employed. The preferred exercise loading method can be set as appropriate depending on the age and health status of the subject, the targeted skeletal muscle, and the amount of skeletal muscle.

Specific examples of exercise loading methods include squatting, knee extension, knee raising, etc. for the quadriceps muscle, one-leg standing exercise, etc. for the gluteus medius muscle, and heel raising exercise, etc. for the gastrocnemius muscle. The above are only examples, and those skilled in the art can adopt an appropriate exercise load method and set the number of times and number of sets according to the target skeletal muscles.

The "physiological muscle stimulation to skeletal muscles" and "vibration stimulation to skeletal muscles" are not limited as long as they can suppress or improve the functional decline of skeletal muscles when used in combination with the muscle function-improving agent of the present invention, and known stimulation means and methods normally used in clinical practice can be employed. A person skilled in the art can set the preferred stimulation load method according to the age and health condition of the subject, the target skeletal muscle, and the amount of skeletal muscle.

The "loading of stimuli to the skeletal muscles with functional decline" may be performed before administration (e.g., 30 minutes before administration, 1 hour before administration, 2 hours before administration, 6 hours before administration, 12 hours before administration, 1 day before administration, etc.), during administration, or after administration (e.g., 30 minutes after administration, 1 hour after administration, 2 hours after administration, 6 hours after administration, 12 hours after administration, 1 day after administration, etc.) of the muscle function-improving agent, as long as the functional decline of skeletal muscle due to aging is suppressed or improved. In the preferable embodiment, the muscle function-improving agent of the present invention can be administered after or simultaneously with stimulus loading to skeletal muscle.

In another aspect, the present invention can be provided as a muscle function-improving agent for suppressing or improving the functional decline of skeletal muscles or for repairing muscle damage of skeletal muscles. In this aspect, the muscle function-improving agent is used in combination with the loading of stimuli to skeletal muscles and contains at least one peptide selected from the following (1) to (3) or a salt thereof as an active ingredient:

(1) peptides consisting of the amino acid sequence shown in SEQ ID No.: 1, and
(2) a fragment of human osteopontin, wherein the C-terminal amino acid sequence of the fragment is the amino acid sequence shown in SEQ ID No.: 1; and
(3) peptides consisting of an amino acid sequence in which one to several amino acids are deleted, substituted, or added in the amino acid sequence of the peptides in (1) or (2) above, and having an inhibitory or improving effect on the functional decline of a skeletal muscle.

In this aspect, the muscle function-improving agent is used in combination with stimulus loading on skeletal muscles to repair skeletal muscle damage, such as muscle tears, muscle atrophy, and muscle degeneration, or to improve skeletal muscle function that is reduced due to such damage, in addition to skeletal muscle with the functional decline due to aging. Skeletal muscle injuries include, for example, muscle tears, muscle atrophy, and muscle degeneration. Specifically, muscle tears associated with major trauma, muscle tears associated with surgery, muscle tears associated with trauma such as fractures, contusions, and separated muscles, muscle injuries in athletes, muscle tears or muscle atrophy after ligament surgery, muscle tears or muscle atrophy after hip replacement surgery, disuse muscle atrophy due to reduced exercise units after head and neck surgery requiring long-term closed mouth conditions, muscle atrophy associated with cancer cachexia, progressive muscle atrophy in inherited neuromuscular diseases such as muscular dystrophy, muscle atrophy associated with erector spinae disorders, muscle atrophy associated with lumbar disc herniation, muscle atrophy associated with dropped head syndrome, inclusion body myositis, fibrosis and scar contracture after surgery involving extensive myectomy, and scar contracture with motor dysfunction after plastic surgery for congenital abnormal muscle morphology such as cleft palate. Scar contractures with motor dysfunction after plastic surgery for congenital muscle morphology abnormalities such as cleft palate, etc. are examples. For example, the muscle function-improving agent can be used in conjunction with exercise loading, such as post-surgical rehabilitation.

The present invention comprises, in another aspect, a muscle fiber hypertrophy-stimulating agent for hypertrophy of fast and/or slow muscle fibers that have atrophied due to aging, comprising at least one peptide selected from the following (1) to (3), or a salt thereof, as an active ingredient:

(1) a peptide consisting of the amino acid sequence shown in SEQ ID No.: 1; and
(2) a fragment of human osteopontin, wherein the C-terminal amino acid sequence of the fragment is the amino acid sequence shown in SEQ ID No.: 1, and
(3) a peptide consisting of an amino acid sequence in which one to several amino acids are deleted, substituted, or added in the amino acid sequence of the peptide in (1) or (2) above, and having the effect of inhibiting or improving the decrease in the number of muscle hygienic cells in skeletal muscle associated with aging.

In another aspect, the present invention provides a muscle regenerative ability-improving agent for improving the decline of muscle regenerative ability of skeletal muscles due to aging, comprising at least one peptide selected from (1) through (3) above or a salt thereof.

The muscle fiber hypertrophy-stimulating agent and the muscle regenerative ability-improving agent of the present invention can be implemented in the same manner as the above-mentioned muscle function-improving agent of the present invention.

The present invention further comprises the following inventions.

(a1) A method for improving the functional decline of a skeletal muscle due to aging, characterized by a step of administering at least one peptide selected from (1) through (3) above or a salt thereof to a subject having a skeletal muscle functionally declined due to aging at a therapeutic effective amount.

(a1') The method for improving the functional decline of a skeletal muscle due to aging according to (a1) above, further comprising a step of applying a stimulus load to the skeletal muscle functionally declined due to aging or the surrounding skeletal muscles.

(a1") The method for improving the functional decline of a skeletal muscle due to aging according to (a1) above, characterized in that the peptide or a salt thereof is administered to the skeletal muscles functionally declined due to aging or the surrounding a skeletal muscle.

(a2) A method of hypertrophy of fast and/or slow muscle fibers atrophied due to aging, characterized by a step of administrating at least one peptide or a salt thereof selected from (1) to (3) above is to a subject having fast and/or slow muscle fibers atrophied by aging at a therapeutically effective amount.

(a2') The method of hypertrophy of fast and/or slow muscle fibers atrophied due to aging according to (a2) above, characterized in that the administration step is performed in combination with stimuli loading to the skeletal muscle.

(b1) At least one peptide selected from (1) to (3) above, or a salt thereof, for use in improving the functional decline of a skeletal muscle due to aging.

(b2) At least one peptide selected from (1) to (3) above, or a salt thereof, for use in hypertrophy of fast and/or slow muscle fibers atrophied due to aging.

(c1) Use of at least one peptide selected from (1) to (3) above or a salt thereof in manufacturing a muscle function-improving agent that inhibits or improves functional decline of a skeletal muscle due to aging.

(c2) Use of at least one peptide selected from (1) to (3) above or a salt thereof in manufacturing a muscle fiber hypertrophy stimulating agent that causes hypertrophy of fast and/or slow muscle fibers atrophied due to aging.

(c1') The peptide or a salt thereof according to (b1) above, wherein the peptide or a salt thereof is used in combination with stimuli loading to the skeletal muscle.

## EXAMPLES

(Example 1)

In this example, a peptide consisting of the amino acid sequence of SVVYGLR (SEQ ID No.: 1) (SV peptide) was administered to aging-accelerated model mice , and their effects on restoring muscle function were confirmed.

SV peptide was synthesized by the Fmoc method using a multi-species solid-phase automated peptide synthesizer (PSSM-8; Shimadzu Corp.) SV peptide was added to PBS and adjusted to 20 ng/ml to prepare an injection formulation.

Aging-accelerated model mice (SAMP10 21 weeks old) were used to perform training on a treadmill (MK-680; Muromachi Kikai Co., Ltd.) and subsequent SV peptide administration according to the schedule in the following table.

Table 2

| Age in weeks | Number of treadmill operations | Training Content |
|---|---|---|
| 21-22 | 2 times/1W | warm up 7m/min (5 minutes) ⇒exercise 17m/min (35 minutes) ⇒cool down 7m/min |
| 23-28 | 1 time/1W | warm up 10m/min (5 minutes) ⇒exercise 18m/min (35 minutes) ⇒cool down 10m/min (5 degree angle) |
| 29 | 1 time/1W | warm up 10m/min (5 minutes) ⇒exercise 18m/min (35 minutes) ⇒cool down 10m/min (5 degree angle)<br>After 10 minutes interval<br>warm up 10m/min (5 minutes) ⇒exercise 18m/min (35 minutes) ⇒cool down 10m/min (5 degree angle) |
| 30-34 | 4 times/1W (Basic training, After 12 hours, After 24 hours, After 72 hours) | (Basic training)<br>warm up 10m/min (5 minutes) =>exercise 18m/min (90 minutes) =>cool down 10m/min (5 degree angle)<br>(Training After 12 hours, Training After 24 hours, Training After 72 hours)<br>warm up 10m/min (5 minutes) =>exercise 18m/min (35 minutes) =>cool down 10m/min (5 degree angle) |
| 35 | 4 times/1W (Basic training, After 12 hours, After 24 hours, After 72 hours) | warm up 10m/min (5 minutes) =>exercise 20m/min (90 minutes) =>cool down 10m/min (5 minutes) (8 degree angle)<br>(Basic training, Training After 12 hours, Training After 24 hours, Training After 72 hours) |

Table 3

| Age in weeks | Dose site and amount (denoted by one-sided dose) | SV concentration |
|---|---|---|
| 21-22 | | |
| 23-28 | Gastrocnemius muscle 0.05 ml (1 time/W) | 20 ng/ml |
| 29 | Gastrocnemius muscle 0.05 ml (1 time/W) | 20 ng/ml |
| 30-34 | Quadriceps muscle (0.04 ml), Hamstring (0.03 ml), Gastrocnemius muscle (0.02 ml), Tibialis anterior muscle (0.01 ml) (2 times/W ; Administer after basic training and training after 72 hours) | 20 ng/ml |
| 35 | Quadriceps muscle (0.04 ml), Hamstring (0.03 ml), Gastrocnemius muscle (0.02 ml), Tibialis anterior muscle (0.01 ml) (2 times/W ; Administer after basic training and training after 72 hours) | 20 ng/ml |

At 30-35 weeks of age, mice underwent the first basic training session and the second to fourth training sessions at 12, 24, and 72 h after the first training session.

Once a week, the mice were tested on a treadmill to measure the number of collisions. After SV peptide injection, the mice have trained on the treadmill again (18 m/min $\times$ 90 min), and the number of times the mice collided with the treadmill wall due to being unable to run at a speed equal to or faster than that of the belt was measured during treadmill training. Treadmill running, peptide injection, and weekly collision measurements were continued until the mice reached 35 weeks of age. In the control group, after training on the treadmill in the same manner as the experimental group, PBS was injected into the bilateral lower limb muscles instead of SV peptide, and the number of collisions was measured once a week.

As a result, although the number of collisions showed an increasing trend over time in the control group after 30 weeks of age, the experimental group showed significant suppression of the increase in the number of collisions compared to the control group (Figure 1). These results suggest that the administration of SV peptide promoted the recovery of muscle function in the lower limbs of the aging accelerated model mice.

(Example2)

In this example, SV peptide was administered to aging-accelerated model mice , and the recovery effect of muscle function after a certain period of time by the administration was confirmed.

Treadmill running was performed using aging-accelerated model mice (SAMP10 36 weeks old) under the same conditions as the first basic training of 35-week-old mice in Example 1, and SV peptide (total 40 ng) was injected into the bilateral lower limb muscles immediately after treadmill running (experimental group).

SV peptide was administered at the same site and dose as in the 35-week-old mice of Example 1. The number of collisions was evaluated by retesting treadmill running (18 m/min × 90 min) at 12, 24, or 72 hours after local injection. In the control group, after treadmill running as in the experimental group, PBS was injected into the bilateral leg muscles instead of SV peptide, and the number of collisions on the treadmill was measured 12, 24, or 72 hours after the injection.

As a result, the number of collisions in the experimental group was significantly reduced at 12, 24, and 72 hours after localization compared to the control group. This result suggests that SV peptide administration promoted the recovery of muscle function after exercise (Figure 2).

(Example 3)

In this example, we evaluated muscle fibers in aging-accelerated model mice after continuous administration of SV peptide.

Aging-accelerated model mice (SAMP10 21 weeks old) were trained on a treadmill (MK-680; Muromachi Kikai Co., Ltd.) (18 m/min × 90 min) and subsequent SV peptide administration was performed according to the same schedule as in Example 1. After 36 weeks of age, training on the treadmill and subsequent SV peptide administration were conducted under the same conditions as at 35 weeks of age, and continued until the aging-accelerated model mice reached 40 weeks of age. In the control group, after training under the same conditions as the experimental group, PBS was injected into the bilateral lower limb muscles instead of SV peptide.

The gastrocnemius and soleus muscles of the lower limbs were then removed from 40-week-old aging-accelerated model mice, sections were prepared according to the standard method, and HE staining was performed. Figure 3A shows a HE-stained image of the gastrocnemius muscle in a transverse section of muscle fibers. Figure 3B shows the mean cross-sectional area of muscle fibers identified as slow muscle in the soleus muscle and the mean cross-sectional area of muscle fibers identified as fast muscle in the gastrocnemius muscle. In the SV-treated group, the mean cross-sectional area of both fast and slow muscle fibers was significantly higher than in the control group (Figure 3B).

(Example 4 )

In this example, we evaluated exercise in aging-accelerated model mice when SV peptide was administered continuously.

Aging-accelerated model mice (SAMP10 28 weeks old) were trained on a treadmill (MK-680; Muromachi Kikai Co., Ltd.) (18 m/min × 90 min) and subsequent SV peptide administration was performed according to the schedule in the following table. After 30 weeks of age, training was performed continuously from Monday to Friday of each week. SV peptides were added to PBS to adjust to a concentration of 1 μg/ml or 20 ng/ml for injection formulation. Localized injections were made into the bilateral leg muscles of each mouse anesthetized by inhalation with sevoflurane immediately after training on Tuesday and Friday (SV group). Exercise evaluations were performed after SV peptide localization on Friday.

Exercise evaluations were conducted for endurance evaluation, in which the belt speed on the treadmill was increased incrementally at regular intervals, and for slope-up evaluation, in which the belt incline was increased incrementally at regular intervals. Figure 4 shows the relationship between the elapsed time and the increase in belt speed or belt incline adopted in the exercise evaluation tests. In the PBS group as the control group, PBS was localized to the bilateral lower limb muscles instead of SV peptide after training on the treadmill in the same manner as in the SV group.

Table 4

| Age in weeks | Number of treadmill operations | Training Content |
|---|---|---|
| 28-29 | 2 times/1W | warm up 7 m/min (5 minutes) ⇒ exercise 17 m/min (35 minutes) ⇒ cool down 7 m/min |

(continued)

| Age in weeks | Number of treadmill operations | Training Content |
|---|---|---|
| After 30 | 5 times/1W (Training Monday ~ Friday) | warm up 10 m/min (5 minutes) ⇒ exercise 18 m/min (35 minutes) ⇒ cool down 10 m/min (5 degree angle) |

Table 5

| Age in weeks | Dose site and amount (denoted by one-sided dose) | SV concentration |
|---|---|---|
| 28-29 | | |
| 30 | Quadriceps muscle (0.06 ml), Hamstring (0.06 ml), Triceps surae muscle (0.03 ml), Tibialis anterior muscle (0.02 ml) (2 times/W Administer after training on Tuesday and Friday) | 1 μg/ml |
| After 31 | Quadriceps muscle (0.15 ml), Hamstring (0.2 ml), Triceps surae muscle (0.1 ml), Tibialis anterior muscle (0.05 ml) (2 times/W Administer after training on Tuesday and Friday) | 20 ng/ml |

The results of the endurance evaluation test are shown in Figure 5 as a graph of the percent change in mean running distance. The change rate of the mean running distance shows the relative value when the mean running distance of each group at 30 weeks (reference date) is set as 100%. The mean running distance for each group was calculated as the mean value of running distance calculated based on the running time and speed of each mouse in each group (the mean distance was calculated in the same manner hereinafter). As shown in Figure 5, in the SV group, the rate of change of the mean running distance increased to about 130%, and the mean running distance increased compared to that at the reference date during the measurement period. On the other hand, in the PBS group, the mean running distance decreased over time without exceeding the mean running distance on the reference day. In the SVN group (SVN group) in which SV peptide was administered at the same site and at the same dose as in the SV group without training on the treadmill, the rate of change in mean running distance was measured. The mean running distance decreased over time without the rate of change in mean running distance exceeding 100% since the reference date (not shown in the figure). Thus, the SV group showed an improvement in motor function that was not observed in the PBS group which received only an exercise stimulus or in the SVN group which received the SV peptide without an exercise stimulus.

The mean workload was calculated as the mean value of workload calculated by multiplying body weight, running time, slope (grade), running speed, and gravitational acceleration $g(9.8 \text{ m/s}^2)$ for each mouse in each group by body weight, running time, slope (grade), running speed, and gravitational acceleration $g(9.8 \text{ m/s}^2)$. As shown in Figure 6, the SV-treated group showed a higher workload relative to the PBS group.

(Example 5)

In this example, the training schedule and SV peptide administration concentration were changed from Example 4, and exercise evaluation (endurance evaluation) was conducted in aging-accelerated model mice when SV peptide was continuously administered. In addition, the cross-sectional area of muscle fibers (gastrocnemius (type I, type IIa, type IIb), extensor digitorum longus, and tibialis anterior muscles) were evaluated in mice that continued training and SV peptide administration until 51 weeks of age.

Aging-accelerated model mice were trained on a treadmill (MK-680; Muromachi Kikai Co., Ltd.) (18 m/min × 90 min) and subsequent SV peptide administration was performed according to the schedule in the following table. 30 weeks of age and thereafter, training was performed continuously from Monday to Friday of each week. SV peptide was added to PBS to adjust to a concentration of 1 μg/ml for an injection formulation and localized to the bilateral leg muscles of each mouse anesthetized by inhalation with sevoflurane immediately after training on Tuesday and Friday (SV group; twice weekly administration). Exercise evaluations were performed every other Friday.

Exercise evaluation was performed by endurance assessment, in which the belt speed on the treadmill was increased stepwise at regular intervals. The elapsed time and belt speed increase used for the endurance evaluation test were the same as in Example 4. In the PBS group as a control group, PBS was injected into the bilateral leg muscles instead of SV peptide after training on the treadmill in the same manner as in the SV group.

Table 6

| Age in weeks | Number of treadmill operations | Training Content |
|---|---|---|
| 28-29 | 2 times/1W | warm up 7 m/min (5 minutes) ⇒ exercise 17 m/min (35 minutes) ⇒ cool down 7 m/min |
| After 30 | 4 times/1W (Training Monday ~ Thursday) | warm up 10 m/min (5 minutes) ⇒ exercise 18 m/min (35 minutes) ⇒ cool down 10 m/min (5 degree angle) |

Table 7

| Age in weeks | Dose site and amount (denoted by one-sided dose) | SV concentration |
|---|---|---|
| After 30 | Quadriceps muscle (0.06 ml), Hamstring (0.06 ml), Triceps surae muscle (0.03 ml), Tibialis anterior muscle (0.02 ml) (2 times/W Administer after training on Tuesday and Friday) | 1 µg/ml |

The results of the endurance evaluation test are shown in Figure 7 as a graph showing the difference between the mean running distance on the reference date (30 weeks of age) and the mean running distance in each test. As shown in Figure 7, the mean running distance of the tests at 31 to 41 weeks of age in the SV group increased from the reference date. The SV group was also able to suppress the decrease in the mean running distance during the test period relative to the PBS group. On the other hand, the PBS group showed a decrease in the mean running distance over time during the test period without exceeding the mean distance traveled on the reference date.

The results of the endurance evaluation test are also shown in Figure 8 as a graph showing the difference between the mean speed on the reference day and the mean speed on each test day. The mean speed for each group was calculated as the mean of the highest speeds reached by each mouse in each group (mean speeds were calculated in the same manner hereinafter). As shown in Figure 8, the mean speed in the SV group increased continuously in the test from 31 to 39 weeks of age. The SV group was also able to suppress the decrease in mean speed during the test period relative to the PBS group. On the other hand, the PBS group showed a significant decrease in mean speed compared to that on the reference date after 43 weeks of age.

The gastrocnemius, extensor digitorum longus, and tibialis anterior muscles of the lower limbs were removed from 51-week-old aging-accelerated model mice, and after sections were prepared according to the standard method, the cross-sectional area of muscle fibers was measured by HE staining. Figure 9 shows the mean cross-sectional area of muscle fibers in each muscle of mice in the SV and PBS groups. The results showed that the mean cross-sectional area of muscle fibers in the gastrocnemius, extensor digitorum longus, and tibialis anterior muscles in the SV group was significantly larger than that in the PBS group. The mean cross-sectional area of muscle fibers was also measured for each type of gastrocnemius muscle fiber (type I, type IIa, and type lib). The mean cross-sectional area of muscle fibers was the mean of more than 50 fibers of each muscle fiber type. The results showed that the cross-sectional area of muscle fibers in the SV group increased compared to the PBS group for type I, type IIa, and type IIb (Figures 10 and 11).

(Example 6 )

In this example, the number of SV peptide doses and dosage in Example 4 was changed as shown in the following table, and exercise evaluation (endurance evaluation) was performed when SV peptide was continuously administered to aging-accelerated model mice. The conditions other than the number of SV peptide doses and dosage were the same as in Example 4.

Specifically, SV peptide was added to PBS to adjust to a concentration of 2 µg/ml for the injectable formulation, and localized to the bilateral lower limb muscles of each mouse anesthetized by inhalation with sevoflurane immediately after training on Friday every other week (even weeks) (SV group; one dose every other week).

Table 8

| Age in weeks | Dose site and amount (denoted by one-sided dose) | SV concentration |
|---|---|---|
| After 30 | Quadriceps muscle (0.06 ml), Hamstring (0.06 ml), Triceps surae muscle (0.03 ml), Tibialis anterior muscle (0.02 ml) (Administer after training on every other Friday (even weeks)) | 2 µg/ml |

The results of the endurance evaluation test are shown in Figure 12 with a graph showing the mean running distance for the SV group (n=3) and the PBS group (n=4). A graph showing the difference between the mean running distance on the reference date (30 weeks old) and the mean running distance in each test is also shown in Figure 13. As shown in Figures 12 and 13, the mean running distance gradually decreased in the PBS group, while the mean running distance increased significantly in the SV group from 31 to 48 weeks of age. Even after 49 weeks of age, the SV group was able to maintain the same mean running distance as the 30-week-old group.

(Example 7)

In this example, the number of SV peptide doses and dosage in Example 4 were changed as shown in the following table, and exercise evaluation (endurance evaluation) was performed when SV peptide was continuously administered to aging-accelerated model mice. The conditions other than the number of SV peptide doses and dosage were the same as in Example 4.

Specifically, SV peptide was added to PBS to adjust to a concentration of 20 µg/ml for the injectable formulation, and localized to the bilateral lower limb muscles of each mouse anesthetized by inhalation with sevoflurane immediately after training once every 4 weeks (Friday) (SV group; once every 4 weeks).

Table 9

| Age in weeks | Dose site and amount (denoted by one-sided dose) | SV concentration |
|---|---|---|
| After 30 | Quadriceps muscle (0.06 ml), Hamstring (0.06 ml), Triceps surae muscle (0.03 ml), Tibialis anterior muscle (0.02 ml) (1 time/4W Administer after training on Friday) | 20 µg/ml |

The results of the endurance evaluation test are shown in Figure 14 with a graph showing the mean running distance for the SV group (n=6) and the PBS group (n=6). In addition, graphs showing the difference between the mean running distance on the reference date (30 weeks of age) and the mean running distance in each test are shown in Figures 15 and 16. As shown in Figures 14-16, the mean running distance of the PBS group decreased after 30 weeks of age, while the mean running distance of the SV group increased significantly from 31 to 36 weeks of age, and even at 41 weeks of age, the mean running distance of the SV group exceeded that of 30 weeks of age. In Figure 16, the sum of the absolute difference in mean running distance relative to the reference date in the SV group and the absolute difference in mean running distance relative to the reference date in the PBS group is shown as the SV treatment effect.

The change over time in the mean maximum speed in each group is shown in Figure 17, and graphs showing the difference between the mean maximum speed on the reference date and the mean maximum speed on each test date are shown in Figures 18 and 19. As shown in Figure 17, the SV group showed a continuous increase in the mean speed during the test from 31 to 36 weeks of age. The SV group also showed the same mean maximum speed at 41 weeks of age as at 30 weeks of age. On the other hand, after 30 weeks of age, the PBS group showed a decrease in the mean maximum speed over time compared to that on the reference day. Thus, the SV group was able to suppress the decrease in the mean maximum speed during the test period compared to the PBS group. In Figure 19, the sum of the absolute difference in mean running distance relative to the reference date in the SV group and the absolute difference in mean running distance relative to the reference date in the PBS group is shown as the SV treatment effect. The treatment effect of more than 0.15 m/sec was confirmed at 34 and 39-41 weeks.

Claims

1. A muscle function-improving agent for inhibiting or improving functional decline of a skeletal muscle due to aging,

comprising the peptide selected from the following (1) to (3) or a salt thereof as an active ingredient:

(1) a peptide consisting of an amino acid sequence shown in SEQ ID No. 1
(2) a fragment of human osteopontin, wherein the C-terminal amino acid sequence of the fragment is the amino acid sequence shown in SEQ ID No. 1, and
(3) a peptide consisting of an amino acid sequence that one to several amino acids are deleted, substituted, or added in the amino acid sequence of the peptide in (1) or (2) above and having the effect of inhibiting or improving the functional decline of the skeletal muscle due to aging.

2. The muscle function-improving agent according to Claim 1,

wherein the peptide consisting of an amino acid sequence that one to several amino acids are deleted, substituted, or added in the amino acid sequence of the peptide in (1) and having the effect of inhibiting or improving the functional decline of the skeletal muscle due to aging is a peptide consisting of the amino acid sequence of any of the following (I) to (IV),
wherein the peptide consisting of an amino acid sequence that one to several amino acids are deleted, substituted, or added in the amino acid sequence of the peptide in (2) and having the effect of inhibiting or improving the functional decline of the skeletal muscle due to aging is a fragment of human osteopontin, the C-terminal amino acid sequence of the fragment being a peptide consisting of the amino acid sequence of any of the following (I) to (IV),

$$X_1-X_2-Val-(Tyr/Phe/Trp)-X_5-X_6-X_7 \quad (I)$$

(In the formula, $X_1$, $X_2$, $X_5$, $X_6$, and $X_7$ represent any amino acid residue, identically or differently)

$$X_1-X_2-Val-(Tyr/Phe/Trp)-X_5-X_6 \quad (II)$$

(In the formula, $X_1$, $X_2$, $X_5$, and $X_6$ represent any amino acid residue, identically or differently)

$$X_2-Val-(Tyr/Phe/Trp)-X_5-X_6-X_7 \quad (III)$$

(In the formula, $X_2$, $X_5$, $X_6$, and $X_7$ represent any amino acid residue, identically or differently)

$$X_1-X_2-Val-(Tyr/Phe/Trp)-X_5-X_6-X_7-X_8 \quad (IV)$$

(In the formula, $X_1$, $X_2$, $X_5$, $X_6$, $X_7$, and $X_8$ represent any amino acid residue, identically or differently) .

3. A muscle atrophy inhibitor for inhibiting or improving muscle atrophy of fast muscle fibers and/or slow muscle fibers due to aging, comprising at least one peptide selected from the following (1) to (3) or a salt thereof as an active ingredient:

(1) a peptide consisting of an amino acid sequence shown in SEQ ID No. 1
(2) a fragment of human osteopontin, wherein the C-terminal amino acid sequence of the fragment is the amino acid sequence shown in SEQ ID No. 1, and
(3) a peptide consisting of an amino acid sequence that one to several amino acids are deleted, substituted, or added in the amino acid sequence of the peptide in (1) or (2) above and having the effect of inhibiting or improving muscle atrophy of fast muscle fibers and/or slow muscle fibers due to aging.

4. The muscle atrophy inhibitor according to Claim 3,

wherein the peptide consisting of an amino acid sequence that one to several amino acids are deleted, substituted, or added in the amino acid sequence of the peptide in (1) and having the effect of inhibiting or improving muscle atrophy of fast muscle fibers and/or slow muscle fibers due to aging is a peptide consisting of the amino acid sequence of any of the following (I) to (IV),

wherein the peptide consisting of an amino acid sequence that one to several amino acids are deleted, substituted, or added in the amino acid sequence of the peptide in (2) and having the effect of inhibiting or improving muscle atrophy of fast muscle fibers and/or slow muscle fibers due to aging is a fragment of human osteopontin, the C-terminal amino acid sequence of the fragment being a peptide consisting of the amino acid sequence of any of the following (I) to (IV),

$$X_1-X_2-Val-(Tyr/Phe/Trp)-X_5-X_6-X_7 \quad (I)$$

(In the formula, $X_1$, $X_2$, $X_5$, $X_6$, and $X_7$ represent any amino acid residue, identically or differently)

$$X_1-X_2-Val-(Tyr/Phe/Trp)-X_5-X_6 \quad (II)$$

(In the formula, $X_1$, $X_2$, $X_5$, and $X_6$ represent any amino acid residue, identically or differently)

$$X_2-Val-(Tyr/Phe/Trp)-X_5-X_6-X_7 \quad (III)$$

(In the formula, $X_2$, $X_5$, $X_6$, and $X_7$ represent any amino acid residue, identically or differently)

$$X_1-X_2-Val-(Tyr/Phe/Trp)-X_5-X_6-X_7-X_8 \quad (IV)$$

(In the formula, $X_1$, $X_2$, $X_5$, $X_6$, $X_7$, and $X_8$ represent any amino acid residue, identically or differently) .

5. The muscle function-improving agent according to Claim 1 or 2,
wherein the agent is for use in combination with the loading of stimuli on the skeletal muscle.

6. The muscle function-improving agent according to Claim 5,
wherein the agent is for preventing or improving the functional decline of the skeletal muscle due to sarcopenia or inclusion body myositis.

7. The muscle function-improving agent according to Claim 6, wherein the loading of the stimulus to the skeletal muscles is exercise therapy.

8. A muscle-function improving agent for inhibiting or improving the functional decline of a skeletal muscle,
wherein the muscle-function improving agent is for use in combination with the loading of stimuli and comprise at least one peptide selected from the following (1) to (3) or a salt thereof as an active ingredient:

(1) a peptide consisting of an amino acid sequence shown in SEQ ID No. 1
(2) a fragment of human osteopontin, wherein the C-terminal amino acid sequence of the fragment is the amino acid sequence shown in SEQ ID No. 1, and
(3) a peptide consisting of an amino acid sequence that one to several amino acids are deleted, substituted, or added in the amino acid sequence of the peptide in (1) or (2) above and having the effect of inhibiting or improving the functional decline of the skeletal muscle.

Fig. 1

Fig. 2

**Fig. 3A**

Gastrocnemius muscle
(PBS)

Gastrocnemius muscle
(SVVYGLR)

**Fig. 3 B**

Muscle fiber cross-sectional area

$(\mu m^2)$

| PBS | SVVYGLR | PBS | SVVYGLR |
| --- | --- | --- | --- |
| Slow muscle | | Fast muscle | |
| (soleus muscle) | | (Gastrocnemius muscle) | |

**Fig.** 4

Endurance evaluation (running distance)          Slope-up evaluation

Endurance evaluation (running distance)          Evaluation with slope

**Fig.** 5

Percentage change in running distance

Fig. 6

(J) Change over time in mean workload

SV ·· ·· PBS

Fig. 7

Difference in running distance relative to reference date

SV PBS

**Fig. 8**

## Difference in maximum speed relative to reference date

SV  PBS

**Fig. 9**

(μm²)

*: p<0.05 **: p<0.01

| SV | PBS | SV | PBS | SV | PBS |

Gastrocnemius muscle | Extensor digitorum longus muscle | Tibialis anterior muscle

**Fig. 10**

**Fig. 11**

PBS Group  Gastrocnemius muscle

SV Group  Gastrocnemius muscle

**Fig. 12**

Average maximum speed between the two groups (change over time)

2,500
2,000
1,500
1,000
500
0

30w 31w 32w 33w 34w 35w 36w 37w 38w 39w 40w 41w 42w 43w 44w 45w 46w 47w 48w 49w 50w

SV · PBS

**Fig. 13**

(m)

Difference in running distance relative to reference date

1400
1200
1000
800
600
400
200
0
-200
-400
-600
-800

31w 32w 33w 34w 35w 36w 37w 38w 39w 40w 41w 42w 43w 44w 45w 46w 47w 48w 49w 50w

☐ PBS  SV  Treatment Effect

**Fig. 14**

(m)

Mean running distance between the two groups (change over time)

**Fig. 15**

(m)

Difference in running distance relative to reference date

* P<0.05,     ** P<0.01,     *** P<0.005

**Fig. 16**

(m)         Difference in running distance relative to reference date

☐ PBS    ▓ SV    ▓ Treatment Effect

**Fig. 17**

(m/s)      Average maximum speed between the two groups (change over time)

━━ SV    ━ ━ PBS

**Fig. 18**

(m/s)      Difference in maximum speed relative to reference date

PBS   SV

\* P<0.05,     \*\* P<0.01,     \*\*\* P<0.005

**Fig. 19**

(m/s)      Difference in maximum speed relative to reference date

PBS   SV   Treatment Effect

## INTERNATIONAL SEARCH REPORT

International application No.

**PCT/JP2021/039133**

### A. CLASSIFICATION OF SUBJECT MATTER

**A61K 38/08**(2019.01)i; **A61K 38/03**(2006.01)i; **A61P 21/00**(2006.01)i; **C07K 7/06**(2006.01)i; **C12N 15/12**(2006.01)i
FI:    A61K38/08; A61P21/00; A61K38/03; C07K7/06; C12N15/12 ZNA

According to International Patent Classification (IPC) or to both national classification and IPC

### B. FIELDS SEARCHED

Minimum documentation searched (classification system followed by classification symbols)

A61K38/08; A61K38/03; A61P21/00; C07K7/06; C12N15/12

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2018/230535 A1 (UNIV OSAKA) 20 December 2018 (2018-12-20) | 1-4 |
|   | claim 1, paragraphs [0012]-[0017], [0023]-[0024], examples 1-4 | |
| Y | | 5-8 |
| X | 北風智也ほか, コラーゲンペプチドの骨格筋における有用性, ビタミン, 2017, vol. 91, pp. 433-436 | 1, 3, 5-8 |
|   | p. 433, left column, line 1 to right column, line 4, p. 434, paragraph [0002], p. 434, right column, lines 18-22, (KITAKAZE, Tomoya et al. The beneficial effect of collagen peptides on skeletal muscle. VITAMINS.) | |
| X | JP 2008-179620 A (LION CORP) 07 August 2008 (2008-08-07) | 1-4 |
|   | claims 1, 6, 7, paragraphs [0009], [0031]-[0034], [0058], table 1 | |
| X | JP 2011-184314 A (MEGMILK SNOW BRAND CO., LTD.) 22 September 2011 (2011-09-22) | 1, 3 |
|   | claim 1, paragraph [0001], test example 2 | |

☑ Further documents are listed in the continuation of Box C.     ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **05 November 2021** | **22 November 2021** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**

| International application No. |
| --- |
| **PCT/JP2021/039133** |

**C.    DOCUMENTS CONSIDERED TO BE RELEVANT**

| Category* | Citation of document, with indication, where appropriate, of the relevant passages | Relevant to claim No. |
| --- | --- | --- |
| Y | 藤巻慎ほか, 運動が骨格筋の肥大とサテライト細胞に与える効果, CLINICAL CALCIUM, 2014, vol. 24, no. 10, pp. 1463-1470<br>p. 1468, left column, the last paragraph to p. 1469, right column, paragraph [0001], (FUJIMAKI, Shin et al.), non-official translation (The effect of exercise on skeletal muscle hypertrophy and satellite cells) | 5-8 |
| Y | 石井直方, サルコペニア　ーそのメカニズムと防止策としての運動, 医学のあゆみ, 2011, vol. 236, no. 5, pp. 519-524<br>p. 522, lines 2-25, p. 523, left column, lines 5 to the last line, (ISHII, Naokata. Journal of clinical and experimental medicine.), non-official translation (Sarcopenia-Its mechanism and exercise as a preventive measure) | 5-8 |
| Y | 木下博ほか, 各種疾患のアンチエイジング療法　筋力低下, 臨牀と研究, 2010, vol. 87, no. 4, pp. 497-503<br>p. 500, right column, line 10 from the bottom to p. 501, left column, line 17, (KINOSHITA, Hiroshi et al. The Japanese Journal of Clinical and Experimental Medicine.), non-official translation (Anti-aging therapy for various diseases: Muscle weakness) | 5-8 |
| Y | 三木屋良輔, 総説　サルコペニアを呈する高齢者への運動効果について, 森ノ宮医療大学紀要, 2017, vol. 11, pp. 3-16<br>p. 9, line 14 to p. 10, p. 11, lines 21-25, (Outline: MIKIYA, Ryosuke. The effectiveness of exercise in the elderly with sarcopenia. Bulletin of Morinomiya University of Medical Sciences.) | 5-8 |

**INTERNATIONAL SEARCH REPORT**

International application No.

**PCT/JP2021/039133**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

INTERNATIONAL SEARCH REPORT
Information on patent family members

International application No.

**PCT/JP2021/039133**

| Patent document cited in search report | | | Publication date (day/month/year) | Patent family member(s) | | | Publication date (day/month/year) |
|---|---|---|---|---|---|---|---|
| WO | 2018/230535 | A1 | 20 December 2018 | US 2020/0206300 A1<br>claim 1, paragraphs [0040]-[0045], [0051]-[0052], examples 1-4 | | | |
| JP | 2008-179620 | A | 07 August 2008 | (Family: none) | | | |
| JP | 2011-184314 | A | 22 September 2011 | US 2012/0329991 A1<br>claim 1, paragraph [0003], test example 2<br>WO 2011/108692 A1<br>EP 2543382 A1<br>TW 201138802 A<br>CN 102781460 A<br>KR 10-2013-0014532 A | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003030925 A
- WO 2008026634 A
- WO 2012172887 A
- WO 2016084935 A
- WO 2018230535 A
- WO 6886559 A

### Non-patent literature cited in the description

- **MACHIDA, S. ; BOOTH, FW.** Regrowth of skeletal muscle atrophied from inactivity. *Med Sci Sports Exerc.,* January 2004, vol. 36 (1), 52-9
- **MIYAGAWA, S. ; SAWA, Y.** Building a new strategy for treating heart failure using Induced Pluripotent Stem Cells. *J Cardiol.,* December 2018, vol. 72 (6), 445-448
- **BAOGE, L. et al.** Treatment of Skeletal Muscle Injury: A Review. *ISRN Orthop.,* 2012, 689012
- **NAKASA, T. et al.** Acceleration of muscle regeneration by local injection of muscle-specific microRNAs in rat skeletal muscle injury model. *J Cell Mol Med.,* October 2010, vol. 14 (10), 2495-505
- **HAMADA, Y.** Angiogenic activity of osteopontin-derived peptide SVVYGLR. *Biochem Biophys Res Commun.,* 10 October 2003, vol. 310 (1), 153-7
- **HAMADA, Y.** Synthetic osteopontin-derived peptide SVVYGLR can induce neovascularization in artificial bone marrow scaffold biomaterials. *Dent Mater J.,* July 2007, vol. 26 (4), 487-92
- **TANAKA, S.** Osteopontin-derived synthetic peptide SVVYGLR has potent utility in the functional regeneration of oral and maxillofacial skeletal muscles. *Peptides,* June 2019, vol. 116, 8-15
- *Jpn J Rehabil Med,* 2017, vol. 54, 764-767